# EUROPEAN PATENT APPLICATION

(11) **EP 4 647 458 A1**
(43) Date of publication of application: **12.11.2025**
(21) Application number: 25173801.9
(22) Date of filing: 30.04.2025
(51) Int. Cl.: C08G 63/547, C08G 63/672, C08G 63/78, C08G 64/42, C08J 11/24, C08G 63/676

(54) **METHOD FOR MANUFACTURING UNSATURATED POLYESTER RESIN, UNSATURATED POLYESTER RESIN AND UNSATURATED POLYESTER CURED PRODUCT**

(30) Priority: 08.05.2024 CN 202410560592
(71) Applicant: Swancor Innovation & Incubation Co., Ltd., Nantou City, Nantou County 540028 (TW)
(72) Inventor: HUANG, Yun-Wen, 540028 Nantou City (TW); WANG, Meng-Wei, 540028 Nantou City (TW)
(74) Representative: Ter Meer Steinmeister & Partner

(57) **Abstract**

A method for manufacturing an unsaturated polyester resin includes alcoholysis step, etherification step, condensation step and dilution step as follows. In alcoholysis step, a polycarbonate, a diol compound and an organic alkaline catalyst are mixed and heated to an alcoholysis temperature to obtain a first mixture. In etherification step, a carbonate compound is added to the first mixture and the first mixture, and the carbonate compound are maintained at an etherification temperature to obtain a second mixture. In the condensation step, the second mixture is cooled and a dianhydride or diacid monomer is added, and the second mixture and the dianhydride or diacid monomer are heated to a condensation temperature to obtain a third mixture. In dilution step, the third mixture is cooled to a dilution temperature, and a styrene or acrylic monomer is added to dilute it to a predetermined solid content to obtain an unsaturated polyester resin.

## Description

### BACKGROUND

### TECHNICAL FIELD

The present invention relates to a method for manufacturing an unsaturated polyester resin. More particularly, the present invention relates to a method for manufacturing an unsaturated polyester resin from polycarbonate, an unsaturated polyester resin and an unsaturated polyester cured product manufactured therefrom.

### DESCRIPTION OF RELATED ART

Unsaturated polyester resin (UPR) is a polymer formed by an esterification of a diacid and a diol and then by a condensation dehydration reaction with an anhydride. This polymer can form a cross-linked network structure at the room temperature by being diluted with a monomer having free radicals and followed by the addition of a peroxide. The main advantages of unsaturated polyester resin are that it is easy to be mixed with fillers, able to cure under the normal temperature and the normal pressure, and has great mechanical strength, weather resistance, water resistance and chemical resistance after curing, which can be widely applied in casting and hand lay-up processes.

Among the prior arts, it has been disclosed that the recycled waste PET is reacted with propylene glycol, ethylene glycol, diethylene glycol, and a catalyst at 210°C for 6 hours. After the reaction is completed, the temperature is reduced to 160°C, and maleic anhydride is added. Then, the temperature is increased to 210°C for another 6 hours, and a polymerization inhibitor and a diluent are added after the reaction is completed to obtain an eco-friendly unsaturated polyester resin, with the recycled PET constitutes approximately 18-25% therein. However, the overall reaction time takes more than 12 hours, which consumes huge amounts of electrical power.

It is an object of the present invention to overcome the drawbacks of the prior art. For example, a process being advantageous in view of required time, energy consumption, eco-friendliness, processibility, feasibility of using of polycarbonate to produce unsaturated polyester resin and chemical and/or mechanical properties of the resulting products (like mechanical strength) should be provided. In this regard, how to produce unsaturated polyester resin from the recycled polymer materials, aimed at shortening the manufacturing time to achieve carbon reduction while maintaining the excellent mechanical strength of the produced unsaturated polyester resin, has become a target of the relevant industries and thus is a particular object of the present invention.

### SUMMARY

A method for manufacturing an unsaturated polyester resin is provided according to one subject of the present invention, which includes steps as follows. An alcoholysis step is performed, an etherification step is performed, a condensation step is performed, and a dilution step is performed. In the alcoholysis step, a polycarbonate, a diol compound and an organic alkaline catalyst are mixed and heated to an alcoholysis temperature to obtain a first mixture. In the etherification step, a carbonate compound is added to the first mixture and the carbonate compound and the first mixture are maintained at an etherification temperature to obtain a second mixture. In the condensation step, the second mixture is cooled and a dianhydride monomer or a diacid monomer is added to the second mixture, and the second mixture and the dianhydride monomer or the diacid monomer are heated to a condensation temperature to obtain a third mixture. In the dilution step, the third mixture is cooled to a dilution temperature, and a styrene monomer or an acrylic monomer is added to the third mixture to dilute the third mixture and the styrene monomer or the acrylic monomer to a predetermined solid content to obtain an unsaturated polyester resin.

An unsaturated polyester resin made from a polycarbonate, e.g. a waste polycarbonate, is provided according to another subject of the present invention, which is obtained by the method for manufacturing the unsaturated polyester resin of the foregoing subject.

An unsaturated polyester cured product made from a polycarbonate, e.g. a waste polycarbonate, is provided according to one another subject of the present invention, which is obtained by performing a curing reaction of the unsaturated polyester resin of the foregoing subject.

### BRIEF DESCRIPTION OF THE DRAWINGS

The present invention can be more fully understood by referring to the following detailed description of the embodiments, with reference made to the accompanying drawings as follows:
Fig. 1 shows a flow chart of a method for manufacturing an unsaturated polyester resin according to an embodiment of the present invention.
Fig. 2 shows a flow chart of a preparation method of the unsaturated polyester cured product according to another embodiment of the present invention.

### DETAILED DESCRIPTION

The present invention will be further exemplified by the following specific embodiments. However, the embodiments can be applied to various inventive concepts and can be embodied in various specific ranges. The specific embodiments are only for the purposes of description and are not limited to these practical details thereof.

### <Method for Manufacturing Unsaturated Polyester Resin>

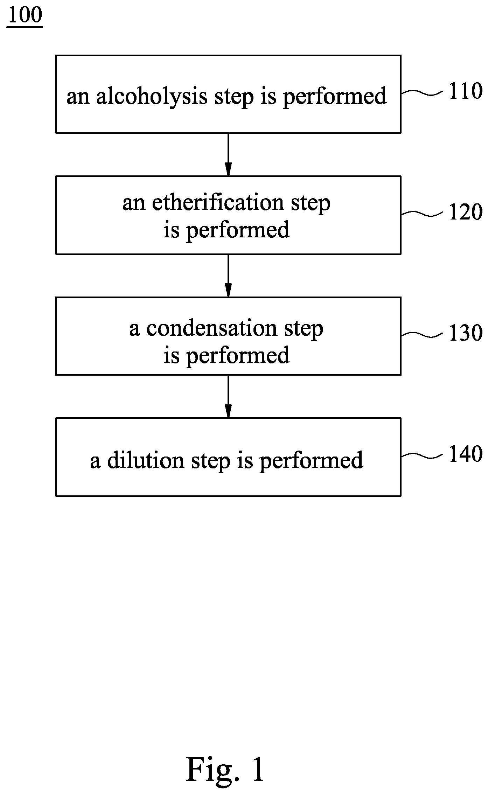

Fig. 1 shows a flow chart of a method for manufacturing an unsaturated polyester resin 100 according to an embodiment of the present invention. In Fig. 1, the method for manufacturing the unsaturated polyester resin 100 includes Step 110, Step 120, Step 130 and Step 140.

Step 110 is an alcoholysis step. Wherein a polycarbonate, a diol compound and an organic alkaline catalyst are mixed and heated to an alcoholysis temperature to obtain a first mixture. Preferably, the polycarbonate has a weight average molecular weight from 5000 to 40000 g/mol, more preferably 8000 to 35000 g/mol. The weight average molecular weight can be determined by gel permeation chromatography. Preferably, the polycarbonate is a bisphenol A-based polycarbonate. Generally, the polycarbonate can be non-waste or waste polycarbonate. Waste polycarbonate is preferred. Preferably, the diol compound has a structure represented by formula (I): 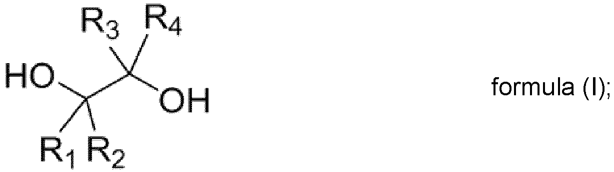 wherein R₁, R₂, R₃ and R₄ are independently a hydrogen atom or an alkyl group with a carbon number of 1 to 6. i.e. with a carbon number of 1, 2, 3, 4, 5 or 6. Preferably R₁, R₂, R₃ and R₄ are independently a hydrogen atom or a methyl group. The organic alkaline catalyst can preferably be 1,8-diazabicyclo[5.4.0]undec-7-ene (DBU), an imidazole-type compound, triethylamine, 1,5,7-triazabicyclo[4.4.0]dec-5-ene (TBD), a pyridine-type compound or a combination thereof. 1,8-diazabicyclo[5.4.0]undec-7-ene (DBU) is a preferred catalyst. Preferably, a molar ratio of the polycarbonate to the diol compound can be 1:1 to 1:2, better can be 1:1 to 1:1.5. The molar amounts of polycarbonate can be calculated based on the molecular weight of repeating units. An adding amount of the organic alkaline catalyst can be 0.2 mol% to 1 mol% of the content of the diol compound, better can be 0.4 mol% to 0.8 mol%. The alcoholysis temperature can be 130°C to 190°C, better can be 140°C to 185 °C, even better can be 150°C to 180°C.

The chemical reaction equation of the alcoholysis step of the present invention is shown in Table 1 below, wherein R₁ to R₄ are defined as above.

| Table 1 |
|---|
| 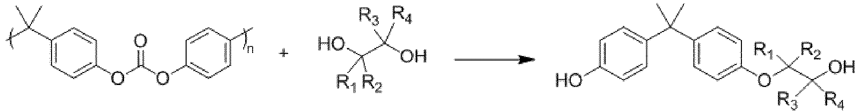 |

Step 120 is an etherification step. Wherein a carbonate compound is added to the first mixture and maintain the first mixture and the carbonate compound at an etherification temperature to obtain a second mixture. Specifically, the carbonate compound has a structure represented by formula (II): 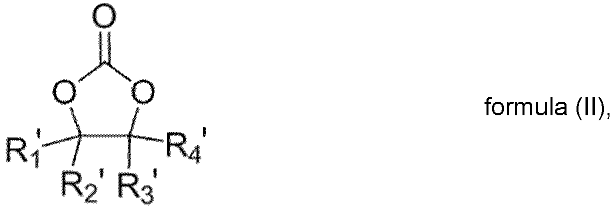 wherein R₁', R₂', R₃' and R₄' are independently a hydrogen atom or an alkyl group with a carbon number of 1 to 6 i.e. with a carbon number of 1, 2, 3, 4, 5 or 6. Preferably R₁', R₂', R₃' and R₄' are independently a hydrogen atom or a methyl group. Specifically, a molar ratio of the carbonate compound to the polycarbonate can be 1.5:1 to 0.8:1, better can be 1.2:1 to 1:1. The etherification temperature can be 130°C to 190°C, better can be 150°C to 190°C, even better can be 170°C to 190°C.

The chemical reaction equation of the etherification step of the present invention is shown in Table 2 below, wherein R₁ to R₄ and R₁' to R₄' are defined as above.

| Table 2 |
|---|
| 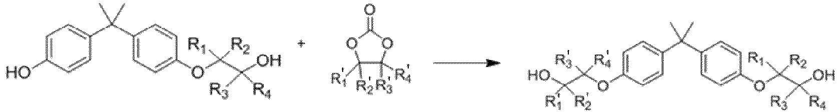 |

Step 130 is a condensation step. Wherein the second mixture is cooled a dianhydride monomer or a diacid monomer is then added to the second mixture, and the second mixture and the dianhydride monomer or the diacid monomer are heated to a condensation temperature to obtain a third mixture. Preferably, the dianhydride monomer can be maleic anhydride, itaconic anhydride, phthalic anhydride or a combination thereof, and the diacid monomer can be maleic acid, fumaric acid, itaconic acid, terephthalic acid, isophthalic acid or a combination thereof. Specifically, a molar ratio of the dianhydride monomer or the diacid monomer to the polycarbonate can be 1:1 to 2:1, better can be 1.4:1 to 1.7:1. The condensation temperature can be 170°C to 230°C, better can be 175°C to 225°C, even better can be 180°C to 220°C.

The chemical reaction equation of the condensation step of the present invention is shown in Table 3 below. Because the dianhydride or diacid monomers can be connected to the hydroxyl groups at either end of the second mixture in the condensation step. The symbol "(')" represents that the third mixtures can be formed by connecting the second mixtures and the dianhydride or diacid monomers in different directions, as shown in Table 3, wherein R₁ to R₄ and R₁' to R₄' are defined as above.

| Table 3 |
|---|
| 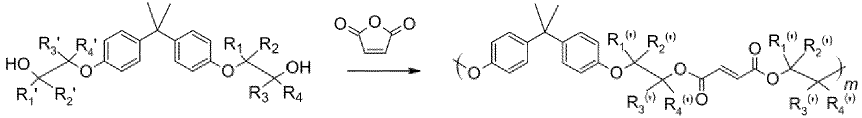 |

Step 140 is a dilution step. Wherein the third mixture is cooled to a dilution temperature, and a styrene monomer or an acrylic monomer is added to the third mixture to dilute it to a predetermined solid content to obtain an unsaturated polyester resin. Specifically, the dilution temperature can be 40°C to 100°C, better can be 40°C to 85°C, even better can be 40°C to 70°C. The predetermined solid content can be 30 wt% to 80 wt%, better can be 40 wt% to 65 wt%.

The present invention further provides an unsaturated polyester resin, which is obtained by the aforementioned method for manufacturing the unsaturated polyester resin 100, wherein a polycarbonate, e.g. a waste polycarbonate, is converted into a diol by alcoholysis, and the diol and an anhydride undergo a dehydration reaction to form the unsaturated polyester resin. The reaction temperature required for the foregoing preparation process is preferably lower than that of the conventional unsaturated polyester resin, and the manufacturing time thereof is shorter.

### <Unsaturated Polyester Cured Product>

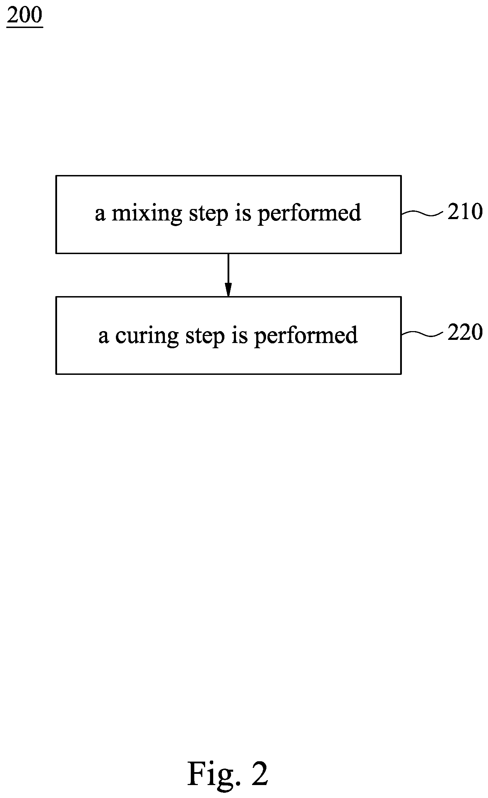

The present invention further provides an unsaturated polyester cured product, which is obtained by performing a curing reaction of the aforementioned unsaturated polyester resin. The aforementioned curing reaction is briefly described with reference to Fig. 2. Fig. 2 shows a flow chart of a preferred preparation method of the unsaturated polyester cured product 200 according to another embodiment of the present invention. In Fig. 2, the preparation method of the unsaturated polyester cured product 200 preferably includes Step 210 and Step 220.

Step 210 is a mixing step. Wherein the aforementioned unsaturated polyester resin can be mixed with a free radical initiator, and a free radical promoter and a free radical inhibitor are added therein to form a curing composition with capability. The free radical promoter and the free radical inhibitor can be used to adjust the reactivity of the free radical initiator at the room temperature, allowing the unsaturated polyester resin to cure at the room temperature while still maintaining a certain capability for further applications.

Step 220 is a curing step. Wherein the aforementioned curing composition can be poured into a mold and stored at the room temperature, e.g. 25 °C, to cure. Then, it is placed into an oven for heating and post-curing to ensure that the unsaturated polyester resin can be completely cured.

The present invention will be further exemplified by the following embodiments so as to facilitate utilizing and practicing the present invention completely by the people skilled in the art without over-interpreting and over-experimenting. However, it is understood that the present invention should not be limited to these practical details thereof, that is, these practical details are used to describe how to implement the materials and methods of the present invention.

### <Examples/Comparative Examples>

### <Preparation of Unsaturated Polyester Resin>

In the preparation method of Example 1, 400.24 g (1.57 moles, calculated based on the molecular weight of repeating units) of polycarbonate particles (purchased from CHIMEI Corporation, and the product code is PC-122) and 146.55 g (2.36 moles) of ethylene glycol (EG) are added to an integrated reaction tank, heated to 160°C, and stirred. When the temperature stabilizes, 1.44 g (0.4 mol% of EG) of 1,8-diazabicyclo[5.4.0]undec-7-ene (DBU) is added to the integrated reaction tank. The integrated reaction tank is then heated to 180°C and maintained at this temperature to react for 2.5 hours to form a first mixture. Then, 166.33 g (1.89 moles) of ethylene carbonate (EC) is added to the first mixture to react at 180°C for another 2.5 hours to form a second mixture. After the reaction is complete, the second mixture is cooled to 120°C, and a distillation apparatus is set up. Then, 231.52 g (2.36 moles) of maleic anhydride (MA) is added to the second mixture, and the initial acid value of the second mixture and the maleic anhydride (MA) is measured to be 170 mg KOH/g. The second mixture and the maleic anhydride (MA) are then heated to 180°C to 215°C to distill off water through the distillation apparatus. When the acid value of the second mixture and the maleic anhydride (MA) drops to 30 mg KOH/g, a third mixture is formed. Finally, the temperature is reduced to 80°C, and 602.07 g of styrene monomer (SM) is added to the third mixture for dilution to a solid content of 55% to obtain the unsaturated polyester resin of Example 1, which has a deep orange-red color.

The preparation method of Example 2 is the same as that of Example 1. The difference is that, the polycarbonate particles in Example 1 are replaced with waste polycarbonate particles (purchased from Yes-Tek Co., Ltd., and the product code is YT-15), and 735.86 g of styrene monomer (SM) is used to dilute the third mixture to a solid content of 50% to obtain the unsaturated polyester resin of Example 2, which has a deep orange-red color.

In the preparation method of Example 3, 400.24 g (1.57 moles) of polycarbonate particles and 96.70 g (1.57 moles) of ethylene glycol (EG) are added to an integrated reaction tank, heated to 160°C, and stirred. When the temperature stabilizes, 0.96 g (0.4 mol% of EG) of DBU is added to the integrated reaction tank. The integrated reaction tank is then heated to 180°C and maintained at this temperature to react for 2.5 hours to form a first mixture. Then, 138.61 g (1.57 moles) of ethylene carbonate (EC) is added to the first mixture to react at 180°C for another 2.5 hours to form a second mixture. After the reaction is complete, the second mixture is cooled to 120°C, and a distillation apparatus is set up. Then, 231.52 g (2.36 moles) of maleic anhydride (MA) is added to the second mixture, and the initial acid value of the second mixture and the maleic anhydride (MA) is measured to be 182 mg KOH/g. The second mixture and the maleic anhydride (MA) are then heated to 180°C to 215°C to distill off water through the distillation apparatus. When the acid value of the second mixture and the maleic anhydride (MA) drops to 30 mg KOH/g, a third mixture is formed. Finally, the temperature is reduced to 80°C, and 700.65 g of styrene monomer (SM) is added to the third mixture for dilution to a solid content of 50% to obtain the unsaturated polyester resin of Example 3, which has a deep orange-red color.

The preparation method of Example 4 is the same as that of Example 3. The difference is that, the amount of maleic anhydride (MA) in Example 3 is changed to 153.95 g (1.57 moles), and 623.08 g of styrene monomer (SM) is used to dilute the third mixture to a solid content of 50% to obtain the unsaturated polyester resin of Example 4, which has an earthy yellow color.

In the preparation method of Example 5, 400.24 g (1.57 moles) of waste polycarbonate fragments (purchased from Super Natural Technology Co., Ltd., and the product code is RPC-Y) and 179.65 g (2.36 moles) of 1,2-propylene glycol (PG) are added to an integrated reaction tank, heated to 160°C, and stirred. When the temperature stabilizes, 2.88 g (0.8 mol% of PG) of DBU is added to the integrated reaction tank. The integrated reaction tank is then heated to 180°C and maintained at this temperature to react for 9.5 hours to form a first mixture. Then, 192.83 g (1.89 moles) of propylene carbonate (PC) is added to the first mixture to react at 180°C for another 9 hours to form a second mixture. After the reaction is complete, the second mixture is cooled to 120°C, and a distillation apparatus is set up. Then, 231.52 g (2.36 moles) of maleic anhydride (MA) is added to the second mixture, and the initial acid value of the second mixture and the maleic anhydride (MA) is measured to be 146.6 mg KOH/g. The second mixture and the maleic anhydride (MA) are then heated to 180°C to 215°C to distill off water through the distillation apparatus. When the acid value of the second mixture and the maleic anhydride (MA) drops to 30 mg KOH/g, a third mixture is formed. Finally, the temperature is reduced to 80°C, and 981.18 g of styrene monomer (SM) is added to the third mixture for dilution to a solid content of 45% to obtain the unsaturated polyester resin of Example 5, which has a deep brown color.

In the preparation method of Example 6, 400.24 g (1.57 moles) of polycarbonate particles and 194.90 g (3.14 moles) of ethylene glycol (EG) are added to an integrated reaction tank, heated to 160°C, and stirred. When the temperature stabilizes, 1.91 g (0.4 mol% of EG) of DBU is added to the integrated reaction tank. The integrated reaction tank is then heated to 180°C and maintained at this temperature to react for 2.5 hours to form a first mixture. Then, 138.61 g (1.57 moles) of ethylene carbonate (EC) is added to the first mixture to react at 180°C for another 2.5 hours to form a second mixture. After the reaction is complete, the second mixture is cooled to 120°C, and a distillation apparatus is set up. Then, 307.91 g (3.14 moles) of maleic anhydride (MA) is added to the second mixture, and the initial acid value of the second mixture and the maleic anhydride (MA) is measured to be 195 mg KOH/g. The second mixture and the maleic anhydride (MA) are then heated to 180°C to 215°C to distill off water through the distillation apparatus. When the acid value of the second mixture and the maleic anhydride (MA) drops to 30 mg KOH/g, a third mixture is formed. Finally, the temperature is reduced to 80°C, and 846.98 g of styrene monomer (SM) is added to the third mixture for dilution to a solid content of 50% to obtain the unsaturated polyester resin of Example 6, which has a deep orange-red color.

In the preparation method of Comparative Example 1, 150 g (0.59 moles) of polycarbonate particles and 72.23 g (1.18 moles) of ethylene glycol (EG) are added to an integrated reaction tank, heated to 160°C, and stirred. When the temperature stabilizes, 0.72 g (0.4 mol% of EG) of DBU is added to the integrated reaction tank. The integrated reaction tank is then heated to 180°C and maintained at this temperature to react for 2.5 hours to form a mixture. After the reaction is complete, the mixture is cooled to 120°C, and a distillation apparatus is set up. Then, 86.77 g (0.88 moles) of maleic anhydride (MA) is added to the mixture, and the initial acid value of the mixture and the maleic anhydride (MA) system is measured to be 175 mg KOH/g. The system is then heated to 180°C to 215°C to distill off water through the distillation apparatus. When the acid value of the whole system drops to 30 mg KOH/g, the temperature thereof is reduced to 80°C. Finally, 272.42 g of styrene monomer (SM) is added to the system for dilution to a solid content of 50% to obtain the unsaturated polyester resin of Comparative Example 1, which has an opaque brown color.

In the preparation method of Comparative Example 2, 400.24 g (1.57 moles) of polycarbonate particles and 146.55 g (2.36 moles) of ethylene glycol (EG) are added to an integrated reaction tank, heated to 160°C, and stirred. When the temperature stabilizes, 1.44 g (0.4 mol% of EG) of DBU is added to the integrated reaction tank. The integrated reaction tank is then heated to 180°C and maintained at this temperature to react for 2.5 hours to form a mixture. After the reaction is complete, the mixture is cooled to 120°C, and a distillation apparatus is set up. Then, 231.52 g (2.36 moles) of maleic anhydride (MA) is added to the mixture, and the initial acid value of the mixture and the maleic anhydride (MA) system is measured to be 186 mg KOH/g. The system is then heated to 180°C to 215°C to distill off water through the distillation apparatus. When the acid value of the whole system drops to 30 mg KOH/g, the temperature thereof is reduced to 80°C. Finally, 666.55 g of styrene monomer (SM) is added to the system for dilution to a solid content of 50% to obtain the unsaturated polyester resin of Comparative Example 2, which has a deep orange-red color.

In the preparation method of Comparative Example 3, 400.24 g (1.57 moles) of polycarbonate particles and 179.65 g (2.36 moles) of propylene glycol (PG) are added to an integrated reaction tank, heated to 160°C, and stirred. When the temperature stabilizes, 1.44 g (0.4 mol% of PG) of DBU is added to the integrated reaction tank. The integrated reaction tank is then heated to 180°C and maintained at this temperature to react for 2.5 hours to form a mixture. After the reaction is complete, the mixture is cooled to 120°C, and a distillation apparatus is set up. Then, 231.52 g (2.36 moles) of maleic anhydride (MA) is added to the mixture, and the initial acid value of the mixture and the maleic anhydride (MA) system is measured to be 178 mg KOH/g. The system is then heated to 180°C to 215°C to distill off water through the distillation apparatus. When the acid value of the whole system drops to 30 mg KOH/g, the temperature thereof is reduced to 80°C. Finally, 699.65 g of styrene monomer (SM) is added to the system for dilution to a solid content of 50% to obtain the unsaturated polyester resin of Comparative Example 3, which has a deep orange-red color.

In the preparation method of Comparative Example 4, 400.24 g (1.57 moles) of waste polycarbonate fragments and 250.55 g (2.36 moles) of diethylene glycol (DEG) are added to an integrated reaction tank, heated to 160°C, and stirred. When the temperature stabilizes, 1.44 g (0.4 mol% of DEG) of DBU is added to the integrated reaction tank. The integrated reaction tank is then heated to 180°C and maintained at this temperature to react for 2 hours to form a mixture. Then, 166.33 g (1.89 moles) of ethylene carbonate (EC) is added to the mixture and reacted at 180°C for another 1.5 hours. After the reaction is complete, the mixture is cooled to 120°C, and a distillation apparatus is set up. Then, 231.52 g (2.36 moles) of maleic anhydride (MA) is added to the mixture, and the initial acid value of the mixture and the maleic anhydride (MA) system is measured to be 139.2 mg KOH/g. The system is then heated to 180°C to 215°C to distill off water through the distillation apparatus. When the acid value of the whole system drops to 30 mg KOH/g, the temperature thereof is reduced to 80°C. Finally, 743.86 g of styrene monomer (SM) is added to the system for dilution to a solid content of 55% to obtain the unsaturated polyester resin of Comparative Example 4, which has a deep brown color.

### <Preparation of Unsaturated Polyester Cured Product>

In the preparation methods of Example 7 to Example 12, 400 g of the unsaturated polyester resins of Example 1 to Example 6 are added to 1.6 g of cobalt octoate and 1.5 g of 2,5-dihydroxy toluene to form the initial mixtures, respectively. After each of the initial mixtures being mixed uniformly, 4.8 g of methyl ethyl ketone peroxide is added to each of the initial mixtures to form curing compositions, respectively. Next, each of the curing compositions is poured into a mold and stored at the room temperature to cure for 1 day. Then, each of the molded compositions is placed into an oven at 105°C to cure for 2 hours to obtain the unsaturated polyester cured products of Example 7 to Example 12.

In the preparation methods of Comparative Example 5 to Comparative Example 7, 400 g of the unsaturated polyester resins of Comparative Example 1 to Comparative Example 3 are added to 1.6 g of cobalt octoate and 1.5 g of 2,5-dihydroxy toluene to form the initial mixtures, respectively. After each of the initial mixtures being mixed uniformly, 4.8 g of methyl ethyl ketone peroxide is added to each of the initial mixtures to form curing compositions, respectively. It could be found that all the initial mixtures could not be cured at the room temperature, and therefore the cured products could not be obtained.

In the preparation method of Comparative Example 8, 400 g of the unsaturated polyester resin of Comparative Example 4 is added to 1.6 g of cobalt octoate to form an initial mixture. However, an exothermic reaction begins during the mixing process and gelation occurs before the peroxide is added, so the cured product could not be obtained.

### <Mechanical and Thermal Properties Evaluation>

The unsaturated polyester cured products of Example 7 to Example 12 are cut for the related mechanical properties measurements such as tensile strength (ASTM D638), bending strength (ASTM D790) and heat deflection (ASTM D648) according to the American Society for Testing and Materials (ASTM) standards. The glass transition temperatures (T_{g}) thereof are measured using a differential scanning calorimetry (DSC) with a heating rate of 10°C/min. The measurement results are shown in Table 1 below.

| Table 1 | | | |
|---|---|---|---|
| | Example 7 | Example 8 | Example 9 |
| Tensile Strength (MPa) | 75.33 | 75.93 | 67.72 |
| Tensile Modulus (GPa) | 3265 | 3205 | 3270 |
| Elongation Rate (%) | 3.81 | 4.12 | 2.76 |
| Bending Strength (MPa) | 128.1 | 137.5 | 102.2 |
| Bending Modulus (GPa) | 3405 | 3316 | 3596 |
| Heat Deflection Temperature (°C) | 100.1 | 104.6 | 96.8 |
| Glass Transition Temperature (°C) | 113.7 | 111.5 | 102.26 |

| | Example 10 | Example 11 | Example 12 |
|---|---|---|---|
| Tensile Strength (MPa) | 84.43 | 53.07 | 53.9 |
| Tensile Modulus (GPa) | 3470 | 3160 | 3065 |
| Elongation Rate (%) | 4.85 | 2.18 | 2.59 |
| Bending Strength (MPa) | 166.5 | 135.88 | 86.3 |
| Bending Modulus (GPa) | 4050 | 3460 | 2850 |
| Heat Deflection Temperature (°C) | 112.0 | 109.8 | 60 |
| Glass Transition Temperature (°C) | 118.12 | 117.4 | 71.4 |

From the results in Table 1, it can be understood that all of the unsaturated polyester resins of Example 1 to Example 6 of the present invention can be successfully processed and molded. In Example 2, the raw material of polycarbonate is replaced with waste polycarbonate, and an unsaturated polyester product with similar properties can also be obtained, which indicates that the manufacturing method of the present invention has great feasibility of reusing of the waste polycarbonate. Furthermore, while the amounts of diol compounds, carbonate compounds and dianhydride monomers in Example 3 to Example 5 are adjusted, the physical properties of the cured products formed thereof can still maintain at a certain level. In Example 6, when the amounts of diol compounds and dianhydride monomers are adjusted to twice of the molar ratio of polycarbonate, the physical properties of the cured product formed thereof become poorer, but are still acceptable in the current composite material industry.

In Comparative Example 1 to Comparative Example 3, no carbonate compound is added during the preparation processes. According to the results of Comparative Example 5 to Comparative Example 7 which are cured therefrom, it can be understood that while lacking of carbonate compounds in the preparation processes, the unsaturated polyester resins formed thereof fail to cure during the curing processes, which shows the importance of adding carbonate compounds in the aforementioned synthetic compositions. Furthermore, comparing Comparative Example 4 to Example 1 to Example 6, in Comparative Example 4, in the manufacturing process, the diol compound is replaced with diethylene glycol (DEG) which has a longer carbon chain, and the unsaturated polyester resin obtained therefrom gelled quickly during the molding process, which is difficult to be handled. Therefore, while the polycarbonate is prepared into the unsaturated polyester resin for reuse, the alcoholysis step and the etherification step are the key influencing factors. In addition, the alcoholysis step and the etherification step in the method for manufacturing the unsaturated polyester resin of the present invention require only a small amount of organic base as the catalyst. The alcoholysis step can be completed and the etherification step can be performed using the carbonate compounds without the need to remove ammonia. Furthermore, the monomer formed therefrom does not require purification or solvent to continue the preparation of the unsaturated polyester resin, while the conventional technique needs to purify and then dissolve the diol compound in a solvent to continue the preparation of the unsaturated polyester resin.

In summary, the waste polycarbonate can be recycled and manufactured into the unsaturated polyester resin in the present invention. Compared with the conventional unsaturated polyester resin manufacturing processes, the required temperature is lower and the manufacturing time is shorter in the manufacturing process thereof, and the cured product obtained therefrom has excellent mechanical strength. In the current trend of environmental carbon reduction, not only can the carbon emissions generated by chemicals of the raw materials be reduced, but the carbon emissions from electricity consumption can also be significantly reduced by shortening time and decreasing temperature in the manufacturing processes, utilizing the manufacturing methods disclosed in the present invention. Thus, dual benefits in reducing carbon emissions can be achieved.

Although the present invention has been described in considerable detail with reference to certain embodiments, it is not limited thereto. Therefore, the spirit and scope of the appended claims should not be limited to the description of the embodiments contained herein. It will be apparent to those skilled in the art that various modifications and variations can be made to the present invention without departing from the scope or spirit of the invention.

## Claims

1. A method for manufacturing an unsaturated polyester resin, comprising:
performing an alcoholysis step, wherein a polycarbonate, a diol compound and an organic alkaline catalyst are mixed and heated to an alcoholysis temperature to obtain a first mixture;
performing an etherification step, wherein a carbonate compound is added to the first mixture, and the first mixture and the carbonate compound are maintained at an etherification temperature to obtain a second mixture;
performing a condensation step, wherein the second mixture is cooled and a dianhydride monomer or a diacid monomer is added to the second mixture, and the second mixture and the dianhydride monomer or the diacid monomer are heated to a condensation temperature to obtain a third mixture; and
performing a dilution step, wherein the third mixture is cooled to a dilution temperature, and a styrene monomer or an acrylic monomer is added to the third mixture to dilute the third mixture and the styrene monomer or the acrylic monomer to a predetermined solid content to obtain an unsaturated polyester resin.

2. The method for manufacturing the unsaturated polyester resin of claim 1, wherein the diol compound has a structure represented by formula (I): 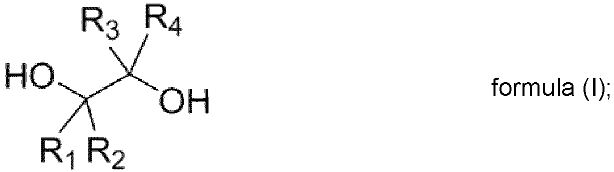 wherein R₁, R₂, R₃ and R₄ are independently a hydrogen atom or an alkyl group with a carbon number of 1 to 6.

3. The method for manufacturing the unsaturated polyester resin of claim 1 or 2, wherein the organic alkaline catalyst is 1,8-diazabicyclo[5.4.0]undec-7-ene, an imidazole-type compound, triethylamine, 1,5,7-triazabicyclo[4.4.0]dec-5-ene, a pyridine-type compound or a combination thereof.

4. The method for manufacturing the unsaturated polyester resin of any one of claims 1 to 3, wherein a molar ratio of the polycarbonate to the diol compound is 1:1 to 1:2.

5. The method for manufacturing the unsaturated polyester resin of any one of claims 1 to 4, wherein an adding amount of the organic alkaline catalyst is 0.2 mol% to 1 mol% of a content of the diol compound.

6. The method for manufacturing the unsaturated polyester resin of any one of claims 1 to 5, wherein the alcoholysis temperature is 130°C to 190°C.

7. The method for manufacturing the unsaturated polyester resin of any one of claims 1 to 6, wherein the carbonate compound has a structure represented by formula (II): 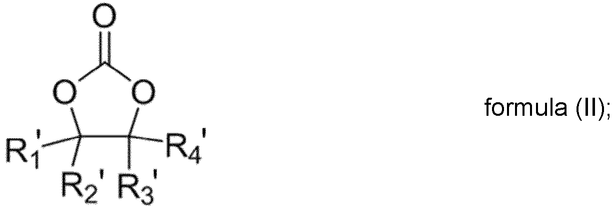 wherein R₁', R₂', R₃' and R₄' are independently a hydrogen atom or an alkyl group with a carbon number of 1 to 6.

8. The method for manufacturing the unsaturated polyester resin of any one of claims 1 to 7, wherein a molar ratio of the carbonate compound to the polycarbonate is 1.5:1 to 0.8:1.

9. The method for manufacturing the unsaturated polyester resin of any one of claims 1 to 8, wherein the etherification temperature is 130°C to 190°C.

10. The method for manufacturing the unsaturated polyester resin of any one of claims 1 to 9, wherein the dianhydride monomer is maleic anhydride, itaconic anhydride, phthalic anhydride or a combination thereof, and/or
wherein the diacid monomer is maleic acid, fumaric acid, itaconic acid, terephthalic acid, isophthalic acid or a combination thereof.

11. The method for manufacturing the unsaturated polyester resin of any one of claims 1 to 10, wherein a molar ratio of the dianhydride monomer or the diacid monomer to the polycarbonate is 1:1 to 2:1.

12. The method for manufacturing the unsaturated polyester resin of any one of claims 1 to 11, wherein the condensation temperature is 170°C to 230°C, and/or, wherein the dilution temperature is 40°C to 100°C.

13. The method for manufacturing the unsaturated polyester resin of any one of claims 1 to 12, wherein the predetermined solid content is 30 wt% to 80 wt%.

14. An unsaturated polyester resin made from a polycarbonate, which is obtainable by the method for manufacturing the unsaturated polyester resin according to any one ofclaims1 to 13.

15. An unsaturated polyester cured product made from a polycarbonate, which is obtainable by performing a curing reaction of the unsaturated polyester resin of claim 14.
